(19) Europäisches Patentamt
European Patent Office
Office européen des brevets

(11) Veröffentlichungsnummer : **0 041 631**
**B1**

(12) # EUROPÄISCHE PATENTSCHRIFT

(45) Veröffentlichungstag der Patentschrift : **01.06.83**

(51) Int. Cl.³ : **C 07 C143/55**, C 07 C 76/02, C 07 B 11/00

(21) Anmeldenummer : **81103745.6**

(22) Anmeldetag : **15.05.81**

(54) **Verfahren zur Herstellung von Nitronaphthalin-sulfonsäuren.**

(30) Priorität : **29.05.80 DE 3020441**

(43) Veröffentlichungstag der Anmeldung : **16.12.81 Patentblatt 81/50**

(45) Bekanntmachung des Hinweises auf die Patenterteilung : **01.06.83 Patentblatt 83/22**

(84) Benannte Vertragsstaaten : **CH DE FR GB IT LI SE**

(56) Entgegenhaltungen :
**EP A 0 003 774**
**DE A 2 837 498**
**HOUBEN-WEYL « Methoden der organischen Chemie » 4. Auflage, Band X/1, 1971, GEORG THIEME VERLAG, Stuttgart Seite 636**

(73) Patentinhaber : **BAYER AG**
**Zentralbereich Patente, Marken und Lizenzen**
**D-5090 Leverkusen 1, Bayerwerk (DE)**

(72) Erfinder : **Behre, Horst, Dr.**
**Zur alten Linde 12**
**D-5068 Odenthal-Eikamp (DE)**
Erfinder : **Blank, Heinz Ulrich, Dr.**
**Am Geusfelde 35**
**D-5068 Odenthal (DE)**
Erfinder : **Burmeister, Gerhard, Dr.**
**Carl-Rumpff-Strasse 45**
**D-5090 Leverkusen (DE)**
Erfinder : **Lindner, Otto, Dr.**
**Strässchen Siefen 31**
**D-5060 Bergisch Gladbach 2 (DE)**

EP 0 041 631 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von Nitronaphthalin-sulfonsäuren

Die Erfindung betrifft ein Verfahren zur Herstellung von Nitronaphthalin-sulfonsäuren durch Nitrieren der entsprechenden Naphthalin-sulfonsäuren.

Es ist bekannt, Nitronaphthalin-sulfonsäuren durch Umsetzen der entsprechenden Naphthalinsulfonsäuren mit Salpetersäure herzustellen (s. z. B. Ullmanns Enzyklopädie der technischen Chemie, 3. Auflage, Band 16, Seite 556, 4. Auflage, Band 17, Seiten 108-115 ; N. Donaldson « The Chemistry and Technology of Naphthalene Compounds », London 1958, Seiten 158-163 ; N.N. Woroshzow « Grundlagen der Synthese von Zwischenprodukten und Farbstoffen », Berlin 1966, Seiten 162-267 ; Houben-Weyl Methoden der organischen Chemie, 4. Auflage (1971), Band 10/1, Seite 636).

Beispielsweise wird die technisch wichtige 1-Nitronaphthalin-3,6,8-trisulfonsäure (Nitro-T-Säure) durch Nitrieren des bei der Naphthalintrisulfierung mit Schwefelsäure und Oleum anfallenden Isomerengemisches von Naphthalintrisulfonsäuren, welches als Hauptbestandteil Naphthalin-1.3.6-trisulfonsäure enthält, in Schwefelsäure als Lösungsmittel mit Salpetersäure hergestellt (s. F.I.A.T., Final Report 1016, Seiten 32-39). Bei dem aus der Literatur bekannten Herstellungsverfahren arbeitet man so, daß man die bei der Naphthalintrisulfierung erhaltene 150 bis 155 °C heiße Lösung der Naphthalintrisulfonsäuren in Schwefelsäure auf 100 °C abkühlt, mit Wasser verdünnt und weiter auf 80 °C abkühlt. Dann überführt man den Ansatz in den Nitrierkessel, kühlt dort auf 35 bis 40 °C ab und nitriert im Verlauf von etwa 10 Stunden mit Mischsäure (86 % Salpetersäure, 12 % Schwefelsäure, 2 % Wasser) bei 35 bis 40 °C.

In der DE-OS 28 37 498 ist ein Verfahren zur Herstellung von 1-Nitronaphthalin-3,6,8-trisulfonsäure aus Naphthalin-1.3.6-trisulfonsäure oder diese enthaltenden Gemischen durch Nitrierung mit Salpetersäure in Schwefelsäure beschrieben, bei dem man zu mindestens 80 % ausreagiertes Nitriergemisch vorlegt, Naphthalin-1.3.6-trisulfonsäure oder diese enthaltende Gemische gelöst in Schwefelsäure zufügt, gleichzeitig 1 bis 1,4 Mol Salpetersäure je Mol Naphthalinsulfonsäure und gegebenenfalls Wasser in einer solchen Weise zufügt, daß die Schwefelsäurekonzentration während der Nitrierung 86 bis 94 Gew.-% beträgt, das Gemisch gut durchmischt und die Reaktionstemperatur bei 30 bis 60 °C hält. Das Verfahren kann diskontinuierlich oder kontinuierlich mit einer Raum/Zeit-Ausbeute von etwa 0,7 bis 1,5 Mol Nitro-T-Säure je Liter Behältervolumen und Stunde durchgeführt werden und liefert bei optimaler Arbeitsweise, d. h. bei diskontinuierlicher Arbeitsweise, wenn man die Reaktionsmischung nach beendeter Zugabe noch 1 bis 2 Stunden bei 35 bis 38 °C nachreagieren läßt, 1-Nitro-naphthalin-3.6.8-trisulfonsäure in einer Ausbeute von 96 %, bezogen auf eingesetzte Naphthalin-1.3.6.-trisulfonsäure.

Die technisch wichtige 1-Nitronaphthalin-4.6.8-trisulfonsäure wird durch Nitrieren des bei der Naphthalintrisulfierung mit Schwefelsäure und Oleum anfallenden Naphthalin-trisulfonsäure-Gemisches, welches als Hauptbestandteil Naphthalin-1.3.5-trisulfonsäure enthält, mit Salpetersäure unter Verwendung von Schwefelsäure als Lösungsmittel hergestellt (s. F.I.A.T. Final Report Nr. 1016, Seiten 42-44). Gemäß diesem Verfahren wird die bei der Naphthalintrisulfierung erhaltene 90 °C heiße Lösung der Naphthalintrisulfonsäuren in Schwefelsäure in den Nitrierkessel übergeführt, nach Abkühlen auf 60 °C mit Wasser verdünnt und bei 30 bis 35 °C mit Mischsäure (86 % Salpetersäure, 12 % Schwefelsäure, 2 % Wasser) nitriert. Die Nitrierung wird 1 Stunde bei 40 °C nachgerührt. Wie die Nacharbeitung des Verfahrens ergeben hat, beträgt die Ausbeute an 1-Nitronaphthalin-4.6.8-trisulfonsäure nur etwa 46 %, bezogen auf eingesetzte Naphthalin-1.3.5-trisulfonsäure (s. Beispiel 16 der vorliegenden Anmeldung).

Von H.E. Fierz-David et. al. (Helv. Chim. Acta 35, (1952), Seiten 2139-2144) wird die Nitrierung von reiner Naphthalin-1.3.5-trisulfonsäure beschrieben. Die Nitrierung ist jedoch mit der Bildung von Neben-Produkten verbunden und liefert deshalb selbst unter günstigsten Bedingungen 1-Nitronaphthalin-4.6.8-trisulfonsäure nur in einer Ausbeute von etwa 48 %.

In der DE-PS 72 665 wird die Nitrierung von 2-Nitronaphthalin-4.8-disulfonsäure mit Salpetersäure in Schwefelsäure bei 30 °C beschrieben. Die angewendete Reaktionszeit beträgt 3 bis 4 Tage. Wie die Nacharbeitung dieses Verfahrens ergeben hat, beträgt die Ausbeute an 1.6-Dinitronaphthalin-4.8-disulfonsäure nur 50 %, bezogen auf eingesetzte 2-Nitronaphthalin-4.8-disulfonsäure (s. Beispiel 19 der vorliegenden Anmeldung).

Gemeinsam ist allen bekannten Verfahren zur Nitrierung von Naphthalin-sulfonsäuren, daß sie entweder niedrige Ausbeuten ergeben und/oder lange Nitrierzeiten erfordern. Wird die Nitrierung wie in der DE-OS 28 37 498 beschrieben, kontinuierlich durchgeführt, so sind relativ lange Nachreaktionszeiten erforderlich, um die höchstmögliche Ausbeute an gewünschter Nitroverbindung zu erhalten. Der Endpunkt der Nitrierreaktion wird dabei in der Weise ermittelt, daß man eine Probe des Nitriergemisches mit Zinkstaub reduziert und den Gehalt der Probe an entstandenen Aminogruppen durch Diazotierung mit Natriumnitrit bestimmt. Die Nitrierreaktion wird beendet, sobald der Verbrauch an Diazotierungsagens nicht mehr ansteigt.

Es wurde nun gefunden, daß man die vorstehend aufgezeigten Nachteile der bekannten Nitrierverfahren, unbefriedigende Ausbeuten und/oder lange Reaktionszeiten und infolgedessen schlechte Raum/Zeit-Ausbeuten, dadurch beseitigen kann, daß man die Nitrierreaktion, nicht mehr wie bislang zu ende führt, sondern vorzeitig abbricht und das Nitriergemisch mit Basen bei einem pH-Wert von 5 bis 14 behandelt. Durch die Kombination dieser beiden Maßnahmen wird eine wesentliche Steigerung der Raum/Zeit-Ausbeuten für alle Nitronaphthalin-sulfonsäuren erreicht. Für die Nitronaphthalin-sulfonsäuren, die bislang nur in unbefriedigenden Ausbeuten hergestellt werden konnten, wird außerdem eine wesentliche Steigerung der Ausbeute erreicht.

Die Erfindung betrifft ein Verfahren zur Herstellung von Nitronaphthalin-sulfonsäuren der Formel (I)

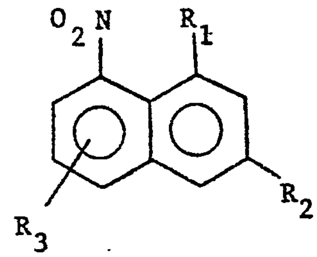

(I)

in der

$R_3$ in 5- oder 6-Stellung steht,

$R_1$ und $R_3$ unabhängig voneinander für eine $SO_3H$- oder $NO_2$-Gruppe stehen,

$R_2$ für ein Wasserstoffatom, eine $SO_3H$- oder $NO_2$-Gruppe steht, mit der Maßgabe, daß mindestens einer der Substituenten $R_1$, $R_2$ oder $R_3$ eine $SO_3H$-Gruppe ist, durch Umsetzen von (Nitro)Naphthalinsulfonsäuren der Formel (II)

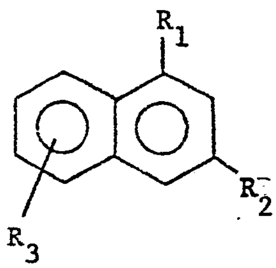

(II)

in der

$R_1$, $R_2$ und $R_3$ die unter Formel (I) angegebene Bedeutung haben, mit Salpetersäure und Aufarbeiten des Nitriergemisches, dadurch gekennzeichnet, daß man die Umsetzung der Naphthalinsulfonsäuren mit Salpetersäure abbricht, wenn mehr als 90 % der eingesetzten Naphthalinsulfonsäuren umgesetzt sind, aber die Nitrierreaktion noch nicht beendet ist und das Nitriergemisch, gegebenenfalls nach Verdünnen mit Wasser, mit Basen bei einem pH-Wert von 5 bis 14 behandelt.

Als Naphthalin-sulfonsäuren der Formel II, die sich besonders für das erfindungsgemäße Verfahren eignen, seien beispielsweise genannt: Naphthalin-1.3.6-trisulfonsäure, Naphthalin-1.3.5-trisulfonsäure, Naphthalin-1.5-disulfonsäure, 2-Nitronaphthalin-4.8-disulfonsäure und 1.5-Dinitronaphthalin-3-sulfonsäure.

In dem erfindungsgemäßen Verfahren kann man sowohl die reinen (Nitro)Naphthalin-sulfonsäuren als auch diese (Nitro)Naphthalin-sulfonsäuren enthaltenden Gemische einsetzen.

Für die erfindungsgemäße Nitrierung der Naphthalin-1.3.6-trisulfonsäure werden vorzugsweise solche Gemische eingesetzt, wie sie bei der Trisulfierung von Naphthalin anfallen. Beispielsweise kann man das Gemisch einsetzen, das bei der Trisulfierung von Naphthalin gemäß F.I.A.T. Final Report 1016, Seiten 23-39 anfällt. Das auf diese Weise hergestellte Gemisch enthält 40 bis 48 Gew.-% Naphthalintrisulfonsäuren in 100 %iger Schwefelsäure. Zusammensetzung dieses Naphthalinsulfonsäuregemisches: 75 Gew.-% Naphthalin-1.3.6-trisulfonsäure, etwa 8 Gew.-% Naphthalin-1.3.5-trisulfonsäure und etwa 12 Gew.-% Naphthalin-1.3.7-trisulfonsäure. Das Sulfierungsgemisch kann außerdem noch geringe Mengen an anderen Naphthalinsulfierungsprodukten und/oder Oxidationsprodukten des Naphthalins enthalten.

Auch auf andere Weise hergestellte Naphthalin-1.3.6-trisulfonsäure-Gemische sind in dem erfindungsgemäßen Verfahren einsetzbar.

Für die erfindungsgemäße Nitrierung der technisch wichtigen Naphthalin-1.3.5-trisulfonsäure werden vorzugsweise Naphthalin-1.3.5-trisulfonsäure enthaltende Gemische, gelöst in Schwefelsäure, eingesetzt, wie sie bei der Trisulfierung von Naphthalin gemäß F.I.A.T. Final Report 1016, Seiten 42-44 anfallen. Diese Gemische enthalten etwa 68 Gew.-% Naphthalin-1.3.5-trisulfonsäure, 21 Gew.-% Naphthalin-1.3.6-trisulfonsäure und 5 % Naphthalin-1.3.7-trisulfonsäure.

Man kann in dem erfindungsgemäßen Verfahren auch auf andere Weise hergestellte Naphthalin-1.3.5-trisulfonsäure-Gemische einsetzen.

Für die Nitrierung der technisch wichtigen 2-Nitronaphthalin-4.8-disulfonsäure kann man 2-Nitronaphthalin-4.8-disulfonsäure enthaltende Gemische, gelöst und/oder suspendiert in Schwefelsäure, einsetzen, wie sie beispielsweise bei der Nitrierung von Naphthalin-1.5-disulfonsäure gemäß dem in der US-PS 2 191 820 beschriebenen Verfahren erhalten werden.

Man kann in dem erfindungsgemäßen Verfahren auch auf andere Weise hergestellte 2-Nitronaphthalin-4.8-disulfonsäure enthaltende Gemische einsetzen.

Es ist ein wesentliches Merkmal des erfindungsgemäßen Verfahrens, daß man die Umsetzung der Naphthalin-sulfonsäuren mit der Salpetersäure bereits vor Beendigung der Nitrierreaktion abbricht. Bei der Nitrierung von Naphthalinsulfonsäuren wird der Fortgang der Nitrierreaktion analytisch verfolgt. Bei den Verfahren gemäß Stand der Technik ist die Nitrierreaktion beendet, wenn der Gehalt des Nitriergemisches an gewünschter Nitronaphthalin-sulfonsäure nicht mehr ansteigt, d. h., wenn die höchstmögliche (= praktisch erreichbare) Ausbeute an Nitronaphthalin-sulfonsäure erreicht wurde.

Erfindungsgemäß wird die Nitrierreaktion aber bereits vor Erreichen dieser höchstmöglichen Ausbeute an gewünschter Nitronaphthalin-sulfonsäure abgebrochen. Und zwar wird erfindungsgemäß die Nitrierreaktion abgebrochen, wenn mehr als 90 % der eingesetzten Naphthalin-sulfonsäure umgesetzt wurde, aber die Ausbeute an gewünschter Nitronaphthalinsulfonsäure erst etwa 90 % der praktisch erreichbaren Ausbeute beträgt. Die praktisch erreichbaren Ausbeuten sind für die einzelnen Nitronaphthalin-sulfonsäuren verschieden. So betragen z. B. die praktisch erreichbaren Ausbeuten für 1-Nitronaphthalin-3.6.8-trisulfonsäure etwa 95 % der theoretischen Ausbeute, für 1-Nitronaphthalin-4.6.8-trisulfonsäure und 1.6-Dinitronaphthalin-4.8-disulfonsäure dagegen nur 50 % der theoretischen Ausbeute.

Deshalb wird die Nitrieraktion beispielsweise bei der Umsetzung von Naphthalin-1.3.6-trisulfonsäure mit Salpetersäure abgebrochen, sobald mehr als 90 % der eingesetzten Naphthalin-1.3.6-trisulfonsäure umgesetzt sind und die Ausbeute an 1-Nitronaphthalin-3.6.8-trisulfonsäure im Nitriergemisch höchstens 90 % bezogen auf umgesetzte Naphthalin-1.3.6-trisulfonsäure beträgt. Vorzugsweise bricht man die Nitrierreaktion ab, sobald mahr als 95 %, insbesondere etwa 98 %, der eingesetzten Naphthalin-1.3.6-trisulfonsäure verbraucht sind und die Ausbeute an 1-Nitronaphthalin-3.6.8-trisulfonsäure im Nitriergemisch höchstens 85 %, vorzugsweise 70 bis 80 %, bezogen auf umgesetzte Naphthalin-1.3.6-trisulfonsäure, beträgt.

Bei der Umsetzung von Naphthalin-1.3.5-trisulfonsäure mit Salpetersäure wird die Nitrierreaktion abgebrochen, sobald mehr als 90 %, vorzugsweise mehr als 93 %, insbesondere etwa 95 bis 98 %, der eingesetzten Naphthalin-1.3.5-trisulfonsäure umgesetzt sind und die Ausbeute an 1-Nitronaphthalin-4.6.8-trisulfonsäure im Nitriergemisch höchstens 50 %, vorzugsweise höchstens 40 %, insbesondere etwa 25 bis 35 %, bezogen auf umgesetzte Naphthalin-1.3.5-trisulfonsäure, beträgt.

Bei der Umsetzung von 2-Nitro-naphthalin-4.8-disulfonsäure mit Salpetersäure bricht man die Nitrierreaktion ab, sobald mehr als 90 %, vorzugsweise mehr als 93 %, insbesondere etwa 95 bis 98 %, der eingesetzten 2-Nitronaphthalin-4.8-disulfonsäure umgesetzt sind und die Ausbeute an 1.6-Dinitronaphthalin-4.8-disulfonsäure höchstens 50 %, vorzugsweise höchstens 40 %, insbesondere etwa 25 bis 35 %, bezogen auf umgesetzte 2-Nitronaphthalin-4.8-disulfonsäure, beträgt.

Die Zusammensetzung der Nitriergemische beim Abbruch der Nitrierreaktion wird wie folgt bestimmt : Das Nitriergemisch wird zunächst unter Kühlen durch Zugabe von Wasser oder Eis verdünnt, die verdünnte Säurelösung, gegebenenfalls durch Einblasen von Luft oder Stickstoff, von nitrosen Gasen befreit und dann mittels Hochdruckflüssigkeitschromatographie unter Verwendung eines sauren Elutionsmittels analysiert.

Die Umsetzung der zu nitrierenden Naphthalin-sulfonsäuren mit Salpetersäure bis zum Abbruch der Nitrierreaktion wird im Falle der Herstellung von Nitronaphthalin-trisulfonsäuren und/oder Nitronaphthalin-disulfonsäuren in 85 bis 95 gew.-%iger, vorzugsweise 88 bis 92 gew.-%iger Schwefelsäure bei Temperaturen von 0 bis 50 °C, vorzugsweise 10 bis 40 °C, durchgeführt. Je Mol zu nitrierender (Nitro)Naphthalin-di(mono)sulfonsäure werden 1,05 bis 2,5 Mol, vorzugsweise 1,15 bis 2,0 Mol, Salpetersäure verwendet.

Im Falle der Herstellung von Nitronaphthalin-disulfonsäuren wird die Umsetzung mit Salpetersäure in 70 bis 90 gew.-%iger, vorzugsweise 75 bis 85 gew.-%iger, Schwefelsäure durchgeführt.

Man kann die Umsetzung der Naphthalinsulfonsäuren mit Salpetersäure auch in überschüssiger 80 bis 100 gew.-%iger, vorzugsweise 90 bis 98 gew.-%iger, Salpetersäure bei Temperaturen von − 40 bis + 20 °C durchführen.

Die Reaktionszeiten bei der Umsetzung von Naphthalinsulfonsäuren mit Salpetersäure bis zum Abbruch der Nitrierreaktion sind abhängig von der Temperatur und/oder der Schwefelsäure-Konzentration oder Salpetersäure-Konzentration (bei Verwendung von Salpetersäure als Lösungsmittel) und/oder dem Salpetersäureüberschuß und/oder der Anfangskonzentration der Naphthalin-sulfonsäuren im Reaktionsgemisch und betragen im allgemeinen etwa einige Minuten bis zu mehreren Stunden. Beispielsweise beträgt die Reaktionszeit bei der Umsetzung von einem Mol Naphthalin-1.3.6-trisulfonsäure mit 1,15 Mol 98 gew.-%iger Salpetersäure in 4,5 Mol 90 gew.-%iger Schwefelsäure bei 30 °C bis zum Abbruch der Nitrierreaktion etwa 20 Minuten. Bei der Umsetzung von einem Mol Naphthalin-1.3.5-trisulfonsäure in 23 Mol 98 gew.-%iger Salpetersäure bei − 20 °C beträgt die Reaktionszeit bis zum Abbruch der Nitrierreaktion etwa 130 Minuten, während bei der Umsetzung von einem Mol 2-Nitronaphthalin-4.8-disulfonsäure mit 2 Mol Salpetersäure in 15 Mol 90 gew.-%iger Schwefelsäure bei 10 °C bis zum Abbruch der Nitrierreaktion eine Reaktionszeit von etwa 3 Stunden erforderlich ist.

Bei der technischen Durchführung des erfindungsgemäßen Verfahrens, insbesondere bei diskontinuierlicher Arbeitsweise, kann es schwierig sein, die kurzen Reaktionszeiten bis zum Abbruch der Nitrierreaktion wegen der hohen abzuführenden Reaktionswärme einzuhalten. In diesem Fall kann es

vorteilhaft sein, die Umsetzung der Naphthalin-sulfonsäuren mit Salpetersäure in Gegenwart von Alkalisulfaten, vorzugsweise Ammoniumsulfat, vorzunehmen. Die Alkalisulfate wirken als Reaktionsverzögerer. Wird die Umsetzung in Schwefelsäure als Lösungsmittel durchgeführt, kann man die Alkalisulfate vor und/oder während der Zugabe der Salpetersäure zusetzen. Man kann die Alkalisulfate jedoch auch in situ im Reaktionsgemisch erzeugen, indem man beispielsweise die Naphthalin-sulfonsäuren in Form ihrer Alkalisalze einsetzt und/oder die Salpetersäure in Form von Alkalinitraten zugibt.

Die Alkalisulfate werden in Mengen von 0,1 bis 1 Mol, vorzugsweise 0,25 bis 0,75 Mol je Sulfogruppe der umzusetzenden Naphthalin-sulfonsäure angewendet.

Das erfindungsgemäße Abbrechen der Nitrierreaktion kann in an sich bekannter Weise, z. B. durch Herabsetzen der Salpetersäure-Konzentration auf eine Konzentration, die zum Nitrieren nicht mehr ausreicht und/oder Abkühlen, vorgenommen werden. Das Herabsetzen der Salpetersäure-Konzentration kann auf verschiedene Weise vorgenommen werden. Üblicherweise geschieht dies durch Verdünnen des Nitriergemisches mit Wasser. Durch Kühlen sorgt man dafür, daß die Temperatur beim Verdünnen 100 °C nicht überschreitet. Das Herabsetzen der Salpetersäurekonzentration kann aber auch durch Abdestillieren von Salpetersäure vorgenommen werden. Das auf diese Weise erhaltene, Reaktionsgemisch wird anschließend der erfindungsgemäßen Behandlung mit Basen bei einem pH-Wert von 5 bis 14 unterworfen.

Das Abbrechen der Nitrierreaktion kann aber auch in der Weise erfolgen, daß man das Nitriergemisch unmittelbar mit den Basen versetzt, mit denen man die erfindungsgemäße Basen-Behandlung durchführen will. D. h. man führt das erfindungsgemäße vorzeitige Abbrechen der Nitrierreaktion und die erfindungsgemäße Behandlung des Nitriergemisches mit Basen in einem einzigen Verfahrensschritt aus.

Als Basen, die in dem erfindungsgemäßen Verfahren eingesetzt werden können, seien beispielsweise genannt: Anorganische Basen wie Alkalihydroxide, -oxide, -carbonate, -hydrogencarbonate, Erdalkalihydroxide, -oxide, -carbonate, -hydrogencarbonate, Ammoniak und organische Basen wie aliphatische, araliphatische, aromatische und heterocyclische Amine. Als aliphatische Amine seien beispielsweise genannt: Methylamin, Triethylamin, Tributylamin, Cyclohexylamin; als araliphatische Amine seien beispielsweise genannt Dimethylbenzylamin; als aromatische Amine seien beispielsweise genannt Dimethylanilin und Diethylanilin; als heterocyclische Amine seien beispielsweise genannt Pyridin, Piperidin und Morpholin.

Bevorzugt verwendet werden aus wirtschaftlichen Gründen die anorganischen Basen, insbesondere Ammoniak.

Die erfindungsgemäße Behandlung der Nitriergemische mit Basen wird bei Temperaturen von − 10 bis + 130 °C, vorzugsweise 10 bis 80 °C, vorgenommen. Der pH-Wert der mit den Basen versetzten wäßrigen Nitriergemische beträgt 5 bis 14, vorzugsweise 5 bis 10.

Die Dauer der Behandlung wird durch analytische Untersuchungen der Reaktionslösungen ermittelt. Die Behandlung wird beendet, sobald der Gehalt der Reaktionslösungen an gewünschter Nitronaphthalinsulfonsäure nicht mehr weiter ansteigt.

Anstelle einer einzigen Base kann man auch verschiedene Basen verwenden und diese verschiedenen Basen stufenweise einsetzen. Zum Beispiel kann man das stark saure Nitriergemisch zunächst in einer ersten Stufe mit billigen anorganischen Basen, z. B. Calciumcarbonat, auf einen pH-Wert von 4 bis 5 bringen und das noch saure Gemisch anschließend, gegebenenfalls nach Entfernen entstandener schwerlöslicher anorganischer Salze (z. B. Gips) mit Ammoniak und/oder einer organischen Base auf den gewünschten pH-Wert zwischen 5 und 14, vorzugsweise 5 und 10, einstellen.

Bei der Nitrierung von Naphthalin-1.3.6-trisulfonsäure hat es sich bewährt, das gegebenenfalls mit Wasser verdünnte Nitriergemisch bei einem pH-Wert von 5 bis 10 und Temperaturen von − 10 bis + 130 °C mit den vorstehend genannten anorganischen und/oder organischen Basen umzusetzen, bis der Gehalt an 1-Nitronaphthalin-3.6.8-trisulfonsäure im Nitriergemisch nicht mehr ansteigt.

Bei der Nitrierung von Naphthalin-1.3.5-trisulfonsäure bzw. 2-Nitronaphthalin-4.8-disulfonsäure hat es sich bewährt, wenn man das gegebenenfalls mit Wasser verdünnte Nitriergemisch bei einem pH-Wert von 5 bis 10 und Temperaturen von − 10 bis + 80 °C mit Ammoniak und/oder den vorstehend genannten organischen Basen behandelt, bis der Gehalt an 1-Nitronaphthalin-4.6.8-trisulfonsäure bzw. 1.6-Dinitronaphthalin-4.8-disulfonsäure im Nitriergemisch nicht mehr ansteigt.

Außer den früher genannten Vorteilen des erfindungsgemäßen Verfahrens, wesentlich erhöhte Raum/Zeit-Ausbeuten und/oder Ausbeuten, weist das erfindungsgemäße Verfahren ferner den Vorteil auf, daß bei ihm der Anfall an nitrosen Gasen wesentlich geringer ist als bei den bekannten Verfahren.

Das erfindungsgemäße Verfahren kann sowohl diskontinuierlich als auch kontinuierlich ausgeführt werden.

Die erfindungsgemäß hergestellten Nitronaphthalin-sulfonsäuren sind wichtige Zwischenprodukte für die Herstellung von Farbstoffen.

Bei den in den nachfolgenden Beispielen verwendeten Prozentangaben handelt es sich um Gewichtsprozent, sofern nichts anderes angegeben ist.

## Beispiel 1

In einem mit 2 Dosiertropftrichtern, Thermometer und Säbelrührer ausgerüsteten 1 l Vierhalskolben

werden 400 g eines Naphthalin-trisulfonsäuregemisches vorgelegt, das wie nachstehend beschrieben erhalten wurde. In das Sulfierungsgemisch werden zunächst unter Kühlen bei 30 °C 40 g Wasser und 100 g 90 %ige Schwefelsäure eingetropft. Anschließend werden unter Kühlen und Rühren 38 g (0,59 Mol) 98 %ige Salpetersäure innerhalb von 5 Minuten eingetropft. Die Reaktionsmischung wird 10 Minuten bei 30 °C nachgerührt. Anschließend wird sie innerhalb von 5 Minuten bei 5 bis 10 °C auf 500 g Eis gegeben. Die entstandene Lösung wird durch Einleiten von Luft von den nitrosen Gasen befreit. Die so erhaltene verdünnte Reaktionslösung wird mit Wasser auf 1 l verdünnt.

Die Zusammensetzung der Lösung wird mittels Hochdruckflüssigkeitschromatographie unter Verwendung eines sauren Elutionsmittels bestimmt. Die gefundenen Werte sind nachstehend angegeben.

| | Gehalt [g/l] | Ausbeute bezogen auf die jeweilige Trisulfonsäure [Mol.-%] |
|---|---|---|
| 1-Nitronaphthalin-3,6,8-trisulfonsäure | 23,6 | 47 |
| 1-Nitronaphthalin-4,6,8-trisulfonsäure | 53,5 | 33,5 |
| Naphthalin-1,3,5-trisulfonsäure | 3,4 | 2,5 |
| Naphthalin-1,3,6-trisulfonsäure | 0,45 | 1,5 |

In einen mit 2 Dosiertropftrichtern, Innenthermometer, pH-Elektrode und Rührer versehenen 3 l Fünfhalskolben läßt man gleichzeitig 1 l der vorstehend beschriebenen verdünnten Nitrierlösung und etwa 720 ml 25 %ige wäßrige Ammoniaklösung unter Kühlen bei 0 bis 5 °C und einem pH-Wert von 9 einlaufen. Die Reaktionslösung wird 2 Stunden bei 20 °C nachgerührt, wobei ihr pH-Wert gegebenenfalls durch Zugabe weiterer Ammoniaklösung auf 9 gehalten wird. Anschließend wird die Lösung mit Wasser auf 2 l verdünnt. Die Hochdruckflüssigkeitschromatographie der so erhaltenen Lösung ergibt folgenden Gehalt an (Nitro-)Naphthalin-trisulfonsäuren :

| | Gehalt [g/l] | Ausbeute bezogen auf die jeweilige Trisulfonsäure [Mol.-%] |
|---|---|---|
| 1-Nitro-naphthalin-3,6,8-trisulfonsäure | 14,8 | 96,5 |
| 1-Nitro-naphthalin-4,6,8-trisulfonsäure | 62,7 | 78,5 |
| Naphthalin-1,3,5-trisulfonsäure | 2,4 | 3,5 |
| Naphthalin-1,3,6-trisulfonsäure | 0,3 | 2,0 |

Das als Ausgangsmaterial verwendete Trisulfierungsgemisch war wie folgt erhalten worden :

In einem 2 l Vierhalskolben, ausgestattet mit einer Dosierschnecke, einem Dosiertropftrichter, Innenthermometer und Säbelrührer, wurden 385 g 100 %ige Schwefelsäure vorgelegt. Über die Dosierschnecke wurden im Verlauf von 30 Minuten unter Rühren 380 g eines Disulfierungsgemisches zugegeben, das folgende Zusammensetzung aufwies :

58 % Naphthalin-1.5-disulfonsäure
7,6 % Naphthalin-1.6-disulfonsäure
3,0 % Naphthalin-1.7-disulfonsäure
2,2 % Naphthalin-1.3.5-trisulfonsäure
4,5 % Naphthalin-1.3.6-trisulfonsäure
0,4 % Naphthalin-1.3.7-trisulfonsäure
24,3 % $SO_3$

und wie folgt erhalten worden war :

Die Lösungen von 128 g Naphthalin (1 Mol) in 640 g Dichlormethan und 243 g $SO_3$ in 950 g Dichlormethan wurden gleichzeitig so schnell zu vorgelegtem Dichlormethan zudosiert, daß die Temperatur zwischen – 5 und – 10 °C gehalten werden konnte. Nach beendeter Zugabe wurde die Reaktionsmischnung 1,5 Stunden bei – 5 bis – 10 °C gehalten und anschließend im Vakuum zur Trockne eingedampft.

Während der Zudosierung des vorstehend beschriebenen Disulfierungsgemisches wurde die Reaktionsmischung auf 70 °C erwärmt. Anschließend wurden über den Dosiertropftrichter 42 g Oleum

(65 %ig) eingetropft. Die Reaktionsmischung wurde 7 Stunden bei 90 °C gerührt.

Die Zusammensetzung des so erhaltenen Trisulfierungsgemisches (Naphthalin-trisulfonsäuregemisches) betrug :

35,5 % Naphthalin-1.3.5-trisulfonsäure
6,8 % Naphthalin-1.3.6-trisulfonsäure
1,3 % Naphthalin-1.3.7-trisulfonsäure
56 % Schwefelsäure

Kohlenstoffgehalt des Sulfierungsgemisches : 14,7 %.

Beispiel 2

40 ml einer Lösung von Naphthalin-1.3.6-trisulfonsäure in 90 %iger Schwefelsäure (Gehalt an Naphthalin-1.3.6-trisulfonsäure : 92 g/l = 0,25 Mol/l) werden unter Kühlen bei 20 °C innerhalb von 10 Minuten mit 11 ml einer Lösung von Salpetersäure in 90 %iger Schwefelsäure (Gehalt der Lösung an Salpetersäure : 63 g/l = 1,0 Mol/l) versetzt. Die Reaktionslösung wird 15 Minuten bei 20 °C nachgerührt, auf 65 g Eis gegeben und im Meßkolben mit Wasser auf 200 ml aufgefüllt. Der Gehalt der so erhaltenen Reaktionslösung an Nitro-naphthalin-trisulfonsäuren wird durch Hochdruckflüssigkeitschromatographie unter Verwendung eines sauren Elutionsmittels bestimmt. Der Gehalt beträgt :

| | Gehalt [g/l] | Ausbeute bezogen auf Naphthalin-1.3.6-trisulfonsäure [Mol.-%] |
|---|---|---|
| Naphthalin-1,3,6-trisulfonsäure | 0,24 | 1,3 |
| 1-Nitronaphthalin-3,6,8-trisulfonsäure | 14,2 | 69 |
| 2-Nitronaphthalin-3,6,8-trisulfonsäure | 0,14 | 0,5 |
| 1-Nitronaphthalin-2,5,7-trisulfonsäure | 0,68 | 3,3 |

50 ml der vorstehend beschriebenen verdünnten Nitrierlösung werden bei 0 °C durch Zugabe von 25 %iger wäßriger Ammoniaklösung auf einen pH-Wert von 9 eingestellt und 2 Stunden auf diesem pH-Wert gehalten. Anschließend wird die Lösung im Meßkolben mit Wasser auf 200 ml aufgefüllt. Die hochdruckflüssigkeitschromatographische Untersuchung der so erhaltenen Lösung ergibt folgenden Gehalt an (Nitro-)Naphthalin-trisulfonsäuren :

| | Gehalt [g/l] | Ausbeute bezogen auf Naphthalin-1.3.6-trisulfonsäure [Mol.-%] |
|---|---|---|
| Naphthalin-1,3,6-trisulfonsäure | 0,06 | 1,3 |
| 1-Nitronaphthalin-3,6,8-trisulfonsäure | 4,95 | 96 |
| 2-Nitronaphthalin-3,6,8-trisulfonsäure | 0,04 | 0,5 |
| 1-Nitronaphthalin-2,5,7-trisulfonsäure | 0,17 | 3,3 |

Beispiel 3

200 ml einer Lösung von Naphthalin-1.3.6-trisulfonsäure in 90 %iger Schwefelsäure (Gehalt der Lösung an Naphthalin-1.3.6-trisulfonsäure : 37,5 g/l = 0,102 Mol/l) werden unter Kühlen bei 0 °C innerhalb von 15 Minuten mit 25 ml einer Lösung von Salpetersäure in 90 %iger Schwefelsäure (Gehalt an Salpetersäure : 63 g/l = 1,0 Mol/l) versetzt. Die Reaktionslösung wird 90 Minuten bei 0 °C nachgerührt, anschließend auf 200 g Eis gegeben und im Meßkolben mit Wasser auf 500 ml aufgefüllt.

Jeweils 50 ml der so erhaltenen verdünnten Nitriergemischlösung werden bei 0 °C mit verschiedenen Basen auf verschiedene pH-Werte eingestellt und nach 2-stündigem Aufbewahren bei diesen pH-Werten mit Wasser auf 500 ml aufgefüllt.

Die Zusammensetzung der so erhaltenen Reaktionslösungen wird mittels Hochdruckflüssigkeitschromatographie unter Verwendung eines sauren Elutionsmittels bestimmt.

Zum Nachweis der Wirkung der Basenbehandlung werden 50 ml der unbehandelten verdünnten

Nitrierlösung mit Wasser auf 500 ml aufgefüllt und der Gehalt der Lösung durch Hochdruckflüssigkeitschromatographie unter Verwendung eines sauren Elutionsmittels bestimmt (Beispiel 3a).

Die Versuchsbedingungen und die Ergebnisse der Versuche sind in der nachstehenden Tabelle 1 zusammengestellt.

Tabelle 1

| Beispiel | Base | pH-Wert | Gehalt [g/l] 1,3,6-SO$_3$H | 1-NO$_2$-3,6,8-SO$_3$H | 2-NO$_2$-3,6,8-SO$_3$H | 1-NO$_2$-2,5,7-SO$_3$H | Ausbeute [Mol.-%] 1,3,6-SO$_3$H | 1-NO$_2$-3,6,8-SO$_3$H | 2-NO$_2$-3,6,8-SO$_3$H | 1-NO$_2$-2,5,7-SO$_3$H |
|---|---|---|---|---|---|---|---|---|---|---|
| 3 a | — | < 1 | 0,046 | 1,102 | 0,008 | 0,034 | 3,0 | 65 | 0,4 | 2,0 |
| b | $NH_3$ | 6 | 0,039 | 1,641 | 0,007 | 0,037 | 2,6 | 97 | 0,5 | 2,2 |
| c | $NH_3$ | 8 | 0,041 | 1,638 | 0,009 | 0,036 | 2,7 | 97 | 0,5 | 2,1 |
| d | $NH_3$ | 10 | 0,038 | 1,586 | 0,007 | 0,030 | 2,5 | 94 | 0,5 | 1,8 |
| e | NaOH | 10 | 0,040 | 1,596 | 0,008 | 0,033 | 2,6 | 94 | 0,5 | 1,9 |
| f | KOH | 10 | 0,044 | 1,645 | 0,009 | 0,037 | 2,9 | 97 | 0,5 | 2,2 |

Beispiel 4

500 ml einer äquimolaren Lösung von Naphthalin-1.3.5-trisulfonsäure und Naphthalin-1.3.6-trisulfonsäure in 90 %iger Schwefelsäure (Gehalt der Lösung : jeweils 36,8 g/l = 0,1 Mol/l 1.3.5- und 1.3.6-Trisulfonsäure) werden unter Kühlen bei 20 °C innerhalb von 15 Minuten mit 120 ml einer Lösung von Salpetersäure in 90 %iger Schwefelsäure (Gehalt an Salpetersäure : 63 g/l = 1,0 Mol/l) versetzt. Die Reaktionslösung wird 45 Minuten bei 20 °C nachgerührt, auf 500 g Eis gegeben und im Meßkolben mit Wasser auf 2 l aufgefüllt.

Jeweils 50 ml der so erhaltenen verdünnten Nitrierlösung werden bei verschiedenen Temperaturen mit wäßrigem Ammoniak auf verschiedene pH-Werte im Bereich von 7 bis 10 eingestellt.

Anschließend wird die Zusammensetzung der so erhaltenen Reaktionslösungen mittels Hochdruckflüssigkeitschromatographie unter Verwendung eines sauren Elutionsmittels bestimmt. Die Versuchsbedingungen und die Ausbeuten an Nitronaphthalin-trisulfonsäuren sind in der nachstehenden Tabelle 2 zusammengestellt (Beispiel 4a ist ein Vergleichsversuch ohne Basenzusatz).

Tabelle 2

| Beispiel | Temperatur °C | pH-Wert | Base | Ausbeute [Mol.-%] 1-NO$_2$-3,6,8-SO$_3$H | 1-NO$_2$-4,6,8-SO$_3$H |
|---|---|---|---|---|---|
| 4 a | 20 | < 1 | — | 77 | 35 |
| b | 0 | 7 | $NH_3$ | 96,5 | 79 |
| c | 0 | 8 | $NH_3$ | 97 | 82,5 |
| d | 0 | 9 | $NH_3$ | 98 | 83 |
| e | 0 | 10 | $NH_3$ | 96,5 | 82,5 |
| f | 20 | 7 | $NH_3$ | 96,5 | 80 |
| g | 40 | 7 | $NH_3$ | 95 | 82,5 |
| h | 60 | 7 | $NH_3$ | 94 | 77,5 |
| i | 20 | 9 | $NH_3$ | 95 | 80,5 |
| k | 40 | 9 | $NH_3$ | 95 | 80,5 |
| l | 60 | 9 | $NH_3$ | 96 | 81 |

(Siehe die Tabelle 3, Seite 9)

Tabelle 3

| Beispiel | Base | Ausbeute [Mol.-%] 1,3,5-$SO_3H$ | 1-$NO_2$-4,6,8-$SO_3H$ | 2-$NO_2$-4,6,8-$SO_3H$ |
|---|---|---|---|---|
| 5 a | — | 2,0 | 28 | 4,1 |
| b | Ammoniak | 2,3 | 75 | 4,2 |
| c | Methylamin | 4,0 | 72 | 5,9 |
| d | Ethylamin | 4,0 | 72 | 6,0 |
| e | Ethylendiamin | 2,9 | 70 | 4,0 |
| f | Dimethylamino-pyridin | 2,5 | 70 | 5,8 |
| g | Ammoniak + 5 ml Pyridin | 1,8 | 85 | 5,9 |

Beispiel 5

200 ml einer Lösung von Naphthalin-1.3.5-trisulfonsäure in 90 %iger Schwefelsäure (Gehalt der Lösung an Naphthalin-1.3.5-trisulfonsäure : 92 g/l = 0,25 Mol/l) werden unter Kühlen bei 20 °C innerhalb von 15 Minuten mit 60 ml einer Lösung von Salpetersäure in 90 %iger Schwefelsäure (Gehalt an Salpetersäure : 75,6 g/l = 1,2 Mol/l) versetzt. Die Reaktionsmischung wird 20 Minuten bei 20 °C nachgerührt, auf 200 g Eis gegeben und im Meßkolben mit Wasser auf 500 ml verdünnt.

Jeweils 50 ml der verdünnten Reaktionslösung werden mit verschiedenen Basen bei 0 °C auf einen pH-Wert von 9 eingestellt. Anschließend wird die Zusammensetzung der Reaktionslösungen mittels Hochdruckflüssigkeitschromatographie unter Verwendung eines sauren Elutionsmittels bestimmt. In der nachstehenden Tabelle 3 sind die verwendeten Basen und die mit ihnen erhaltenen Ausbeuten zusammengestellt. Beispiel 5a ist ein Vergleichsversuch ohne Basenzusatz.

Beispiel 6

50 ml einer Lösung von 2-Nitronaphthalin-4.8-disulfonsäure (Nitro-Armstrongsäure) in 90 %iger Schwefelsäure (Gehalt an Nitro-Armstrongsäure : 16,65 g/l = 0,05 Mol/l) werden unter Kühlen bei 20 °C innerhalb von 15 Minuten mit 11 ml einer Lösung von Salpetersäure in 90 %iger Schwefelsäure (Gehalt an Salpetersäure : 31,5 g/l = 0,5 Mol/l) versetzt. Die Reaktionsmischung wird 1 Stunde bei 20 °C gerührt, anschließend auf 100 g Eis gegeben und mit Wasser auf 250 ml aufgefüllt.

50 ml der verdünnten sauren Nitrierlösung werden bei 0 °C mit 25 %iger wäßriger Ammoniaklösung auf einen pH-Wert von 9 eingestellt und 2 Stunden bei 20 °C auf diesem pH-Wert gehalten.

Die Zusammensetzung der so erhaltenen Reaktionslösungen wird mittels Hochdruckflüssigkeitschromatographie unter Verwendung eines sauren Elutionsmittels bestimmt.

Nachstehend sind die Ausbeuten an Nitronaphthalin-sulfonsäuren in der sauren Reaktionslösung und in der mit Ammoniak behandelten Reaktionslösung einander gegenübergestellt.

| Reaktionsprodukte | Ausbeute saure Reaktions-lösung | (Mol.-%) mit Ammoniak behandelte Reaktionslösung |
|---|---|---|
| 1,6-Dinitronaphthalin-4,8-disulfonsäure | 30 | 89 |
| 2,6-Dinitronaphthalin-4,8-disulfonsäure | 5,4 | 5,8 |
| 2-Nitronaphthalin-4,8-disulfonsäure | 5,5 | 5,8 |

Beispiel 7

Die Nitrierung der 2-Nitro-naphthalin-4.8-disulfonsäure wird wie in Beispiel 6 beschrieben vorgenommen, nur werden bei den einzelnen Versuchen der Salpetersäure-Überschuß, die Reaktionstemperatur und die Nachreaktionszeit verändert. In der nachstehenden Tabelle sind die bei den einzelnen Versuchen angewendeten Bedingungen und die unter diesen Bedingungen erhaltenen Ergebnisse zusammengestellt.

Tabelle 4

| Beispiel | $HNO_3$-Überschuß (%) | Reaktions-temperatur (°C) | Nachreak-tionszeit (h) | Base | Ausbeute [Mol.-%] 1,6-$NO_2$-4,8-$SO_3H$ | 2,6-$NO_2$-4,8-$SO_3H$ | 2-$NO_2$-4,8-$SO_3H$ |
|---|---|---|---|---|---|---|---|
| 7 a | 150 | 20 | 1 | keine | 29,5 | 5,9 | 4,3 |
| | | | | Ammoniak | 87 | 5,7 | 4,3 |
| b | 200 | 20 | 1 | keine | 30 | 5,7 | 2,5 |
| | | | | Ammoniak | 85 | 5,4 | 2,5 |
| c | 100 | 20 | 2 | keine | 29 | 6,4 | 3,3 |
| | | | | Ammoniak | 89 | 6,0 | 3,3 |
| d | 100 | 40 | 1 | keine | 46,5 | 5,7 | 4,2 |
| | | | | Ammoniak | 77 | 5,5 | 5,4 |

Beispiel 8

In einem mit Dosiertropftrichter, Innenthermometer und Säbelrührer ausgestatteten 1 l Dreihalskolben wird eine Suspension von 184 g (0,5 mol) Naphthalin-1.3.6-trisulfonsäure in 90 %iger Schwefelsäure vorgelegt. Die Suspension wird unter Rühren innerhalb von 10 Minuten bei 30 °C tropfenweise mit 37 g (0,575 Mol) 98 %iger Salpetersäure versetzt. Die Reaktionsmischung wird 10 Minuten bei 30 °C nachgerührt und anschließend bei einer 40 °C nicht überschreitenden Temperatur in 425 g Eiswasser eingetragen. Nach dem Austreiben der nitrosen Gase durch Einleiten von Luft wird die Reaktionslösung mit Wasser auf 1 l aufgefüllt. Die Zusammensetzung der so erhaltenen Nitrierlösung wird mittels Hochdruckflüssigkeitschromatographie unter Verwendung eines sauren Elutionsmittels bestimmt. Die Lösung weist folgenden Gehalt an (Nitro-)Naphthalin-sulfonsäuren auf :

| | Gehalt (g/l) | Ausbeute bezogen auf Naphthalin-1,3,6-trisulfonsäure (Mol.-%) |
|---|---|---|
| 1-Nitronaphthalin-3,6,8-trisulfonsäure | 161,0 | 78 |
| 2-Nitronaphthalin-3,6,8-trisulfonsäure | 1,0 | 0,5 |
| 1-Nitronaphthalin-2,5,7-trisulfonsäure | 8,3 | 4,0 |
| Naphthalin-1,3,6-trisulfonsäure | 0,7 | 0,4 |

In eine Lösung von 106,5 g (0,75 Mol) Natriumsulfat in 500 ml Wasser läßt man bei 60 bis 70 °C unter Einhaltung eines pH-Wertes von 3 bis 4 gleichzeitig 1 000 ml der vorstehend beschriebenen sauren verdünnten Nitriergemischlösung und eine etwa 50 %ige wäßrige Calciumcarbonat-Suspension einlaufen. Die so erhaltene Mischung wird 30 Minuten bei 60 bis 70 °C nachgerührt. Dann wird der ausgefallenen Gips abfiltriert und mit warmem Wasser gewaschen bis der Ablauf farblos ist.

Filtrat und Ablauf werden vereint und durch Zugabe von etwa 10 ml gesättigter wäßriger Natriumcarbonatlösung bei 20 °C auf einen pH-Wert von 9 eingestellt. Das ausgefallene Calciumcarbonat wird abfiltriert und mit warmem Wasser gewaschen. Filtrat und Ablauf werden vereinigt und mit Wasser auf 2 l aufgefüllt. Die Zusammensetzung der so hergestellten Lösung wird mittels Hochdruckflüssigkeitschromatographie unter Verwendung eines sauren Elutionsmittels bestimmt. Die Lösung weist folgenden Gehalt an Nitro-naphthalin-sulfonsäuren auf :

| | Gehalt (g/l) | Ausbeute bezogen auf Naphthalin-1,3,6-trisulfonsäure (Mol.-%) |
|---|---|---|
| 1-Nitronaphthalin-3,6,8-trisulfonsäure | 104,3 | 96 |
| 2-Nitronaphthalin-3,6,8-trisulfonsäure | 0,6 | 0,5 |
| 1-Nitronaphthalin-2,5,7-trisulfonsäure | 4,0 | 3,7 |
| Naphthalin-1,3,6-trisulfonsäure | 0,27 | 0,3 |

Beispiel 9

In der in Beispiel 1 beschriebenen Nitrier-Apparatur werden 397 g eines Trisulfierungsgemisches vorgelegt, das gemäß dem in der DE-OS 27 18 207 beschriebenen Verfahren erhalten worden war. Dieses Gemisch wies folgende Zusammensetzung auf :

3,7 % Naphthalin-1.3.5-trisulfonsäure
36,2 % Naphthalin-1.3.6-trisulfonsäure
5,6 % Naphthalin-1.3.7-trisulfonsäure
55 % Schwefelsäure
Kohlenstoffgehalt des Sulfierungsgemisches : 15,1 %.

In dieses Trisulfierungsgemisch werden zunächst unter Kühlen bei 30 °C 35 g Wasser und 50 g 90 %ige Schwefelsäure eingetropft. Anschließend werden unter Kühlen und Rühren 39 g (0,6 Mol) 98 %ige Salpetersäure innerhalb von 10 Minuten eingetropft. Die Reaktionsmischung wird 10 Minuten bei 30 °C nachgerührt. Anschließend wird die Reaktionsmischung in 450 g Eiswasser eingetragen, wobei eine Temperatur von 30 °C nicht überschritten wird. Die nitrosen Gase werden durch Einleiten von Luft aus der entstandenen Lösung ausgetrieben. Die verdünnte Reaktionslösung wird mit Wasser auf 1 l aufgefüllt.

Die Zusammensetzung der so erhaltenen verdünnten Nitriergemischlösung wird mittels Hochdruckflüssigkeitschromatographie unter Verwendung eines sauren Elutionsmittels bestimmt. Die Lösung weist folgenden Gehalt an Nitronaphthalin-sulfonsäuren auf :

| | Gehalt (g/l) | Ausbeute bezogen auf die jeweilige Naphthalintrisulfonsäure (Mol.-%) |
|---|---|---|
| 1-Nitronaphthalin-3,6,8-trisulfonsäure | 129,2 | 80,0 |
| 2-Nitronaphthalin-3,6,8-trisulfonsäure | 0,32 | 0,2 |
| 1-Nitronaphthalin-2,5,7-trisulfonsäure | 5,5 | 3,4 |
| Naphthalin-1,3,6-trisulfonsäure | 0,43 | 0,3 |
| 1-Nitronaphthalin-4,6,8-trisulfonsäure | 6,0 | 36,5 |
| 2-Nitronaphthalin-4,6,8-trisulfonsäure | 0,91 | 5,5 |
| Naphthalin-1,3,5-trisulfonsäure | 0,37 | 2,5 |
| 1-Nitronaphthalin-3,5,7-trisulfonsäure | 11,4 | 45,5 |
| 2-Nitronaphthalin-3,5,7-trisulfonsäure | 1,7 | 7,0 |

In 100 ml Wasser läßt man unter Kühlen und Rühren bei 0 bis 5 °C und unter Einhaltung eines pH-Wertes von 9 gleichzeitig 500 ml der vorstehend beschriebenen verdünnten sauren Nitriergemischlösung und etwa 350 ml 25 %ige wäßrige Ammoniaklösung einlaufen. Nach 2-stündigem Nachrühren bei 20 °C unter Konstanthalten des pH-Wertes wird die Reaktionslösung mit Wasser auf 1 l aufgefüllt.

Die Zusammensetzung der so erhaltenen Reaktionslösung wird mittels Hochdruckflüssigkeitschromatographie unter Verwendung eines sauren Elutionsmittels bestimmt. Die Lösung weist folgenden Gehalt an Nitronaphthalinsulfonsäuren auf :

| | Gehalt (g/l) | Ausbeute bezogen auf die jeweilige Naphthalin-trisulfonsäure (Mol.-%) |
|---|---|---|
| 1-Nitronaphthalin-3,6,8-trisulfonsäure | 77,9 | 96,5 |
| 2-Nitronaphthalin-3,6,8-trisulfonsäure | 0,24 | 0,3 |
| 1-Nitronaphthalin-2,5,7-trisulfonsäure | 2,4 | 3,0 |
| Naphthalin-1,3,6-trisulfonsäure | 0,29 | 0,4 |
| 1-Nitronaphthalin-4,6,8-trisulfonsäure | 6,6 | 79,5 |
| 2-Nitronaphthalin-4,6,8-trisulfonsäure | 0,45 | 5,5 |
| Naphthalin-1,3,5-trisulfonsäure | 0,22 | 3,0 |

(Fortsetzung)

| | Gehalt (g/l) | Ausbeute bezogen auf die jeweilige Naphthalintrisulfonsäure (Mol.-%) |
|---|---|---|
| 1-Nitronaphthalin-3,5,7-trisulfonsäure | 5,7 | 46,0 |
| 2-Nitronaphthalin-3,5,7-trisulfonsäure | 0,94 | 7,5 |

Beispiel 10

In der in Beispiel 1 beschriebenen Nitrierapparatur werden 400 g eines Naphthalin-trisulfierungsgemisches vorgelegt, das wie in Beispiel 1 beschrieben erhalten worden war. Das Gemisch wies folgende Zusammensetzung auf:

36,7 % Naphthalin-1.3.5-trisulfonsäure
7,5 % Naphthalin-1.3.6-trisulfonsäure
1,4 % Naphthalin-1.3.7-trisulfonsäure
54 % Schwefelsäure
Kohlenstoffgehalt des Sulfierungsgemisches: 15,2 %.

In das Trisulfierungsgemisch werden zunächst unter Kühlen und Rühren bei 30 °C 40 g Wasser und 200 g 90 %ige Schwefelsäure eingetropft und außerdem 99 g (0,75 Mol) Ammoniumsulfat eingetragen. Anschließend werden unter Kühlen und Rühren 48 g (0,75 Mol) 98 %ige Salpetersäure innerhalb von 5 Minuten bei 30 °C eingetropft. Die Reaktionsmischung wird 60 Minuten bei 30 °C nachgerührt. Anschließend wird das Nitriergemisch wie in Beispiel 1 beschrieben durch Verdünnen mit Wasser und Behandeln mit Ammoniak aufgearbeitet. Gemäß Hochdruckflüssigkeitschromatographie weist die so erhaltene wäßrige verdünnte Lösung folgende Gehalte an (Nitro-)Naphthalin-sulfonsäuren auf (zum Nachweis der ausbeutesteigernden Wirkung des Basenzusatzes bei der Aufarbeitung des Nitriergemisches wurde ein Teil der wäßrigen verdünnten Nitriergemischlösung ohne Ammoniakzusatz aufgearbeitet):

| | Base | Ausbeute bezogen auf jeweilige Naphthalin-trisulfonsäure (Mol.-%) |
|---|---|---|
| 1-Nitronaphthalin-3,6,8-trisulfonsäure | keine | 63 |
| | Ammoniak | 90 |
| 1-Nitronaphthalin-4,6,8-trisulfonsäure | keine | 37,5 |
| | Ammoniak | 76,5 |
| Naphthalin-1,3,5-trisulfonsäure | keine | 4,3 |
| | Ammoniak | 4,3 |
| Naphthalin-1,3,6-trisulfonsäure | keine | 2,1 |
| | Ammoniak | 2,3 |

Beispiel 11

Das in Beispiel 1 beschriebene Naphthalintrisulfierungsgemisch wird unter den in Beispiel 1 beschriebenen Bedingungen nitriert. Das auf diese Weise erhaltene Nitriergemisch wird jedoch dann nicht verdünnt, sondern unverdünnt gleichzeitig mit 25 %iger wäßriger Ammoniaklösung unter Kühlen in 500 g Eiswasser eingetragen unter Einhaltung eines pH-Wertes von 9.

Die Zusammensetzung der so erhaltenen wäßrigen Reaktionslösung wird mittels Hochdruckflüssigkeitschromatographie unter Verwendung eines sauren Elutionsmittels bestimmt. Der Gehalt der Lösung an (Nitro-)Naphthalin-sulfonsäuren beträgt:

(Siehe die Tabelle, Seite 13)

| | Base | Ausbeute bezogen auf die jeweilige Naphthalin-trisulfonsäure (Mol.-%) |
|---|---|---|
| 1-Nitronaphthalin-3,6,8-trisulfonsäure | Ammoniak | 96,5 |
| 1-Nitronaphthalin-4,6,8-trisulfonsäure | Ammoniak | 76,0 |
| Naphthalin-1,3,5-trisulfonsäure | Ammoniak | 4,0 |
| Naphthalin-1,3,6-trisulfonsäure | Ammoniak | 1,5 |

Beispiel 12

In einer Nitrierapparatur werden 381 g eines Trisulfierungsgemisches vorgelegt, das gemäß dem in F.I.A.T. Final Report n° 1016, S. 42-44 beschriebenen Verfahren erhalten wurde. Das Trisulfierungsgemisch wies folgende Zusammensetzung auf :

34,7 % Naphthalin-1.3.5-trisulfonsäure
11,0 % Naphthalin-1.3.6-trisulfonsäure
2,15 % Naphthalin-1.3.7-trisulfonsäure
Rest Schwefelsäure und freies $SO_3$
Kohlenstoffgehalt :
15,8 %.

In dieses Sulfierungsgemisch werden zunächst unter Kühlen bei 35 °C 15 g Wasser eingetropft und außerdem 33 g (0,25 Mol) Ammoniumsulfat eingetragen. Anschließend werden unter Kühlen und Rühren 48 g (0,75 Mol) 98 %ige Salpetersäure innerhalb von 23 Minuten bei 35 °C eingetropft. Die Reaktionsmischung wird 67 Minuten bei 35 °C nachgerührt. Anschließend wird das Nitriergemisch wie in Beispiel 1 beschrieben durch Verdünnen mit Wasser und Behandeln mit Ammoniak aufgearbeitet.

Die Ausbeuten an Nitronaphthalinsulfonsäuren werden durch Hochdruckflüssigkeitschromatographie unter Verwendung eines sauren Elutionsmittels bestimmt. Zum Vergleich werden auch die Ausbeuten in der sauren, nicht mit Ammoniak behandelten, Nitriergemischlösung bestimmt. Die Ausbeuten sind nachstehend angegeben :

| | Base | Ausbeute bezogen auf jeweilige Naphthalin-trisulfonsäure (Mol.-%) |
|---|---|---|
| 1-Nitronaphthalin-3,6,8-trisulfonsäure | keine | 81,2 |
| | Ammoniak | 94,7 |
| 1-Nitronaphthalin-4,6,8-trisulfonsäure | keine | 42,9 |
| | Ammoniak | 71,9 |
| Naphthalin-1,3,5-trisulfonsäure | keine | 1,4 |
| | Ammoniak | 2,4 |
| Naphthalin-1,3,6-trisulfonsäure | keine | 0,34 |
| | Ammoniak | 0,38 |

Beispiel 13

Eine gemäß Beispiel 12 hergestellte, verdünnte saure Nitriergemischlösung wird gleichzeitig mit einer wäßrigen Calciumcarbonat-Suspension bei 70 °C unter Einhaltung eines pH-Wertes von 2 in eine Vorlage gegeben. Die so erhaltene Reaktionsmischung wird 30 Minuten bei 70 °C nachgerührt. Der pH-Wert wird gegebenenfalls durch Zugabe von etwas Schwefelsäure auf 2 gehalten. Nach dem Abfiltrieren des ausgefallenen Gipses und Waschen mit warmem Wasser läßt man die vereinigten Filtrate gleichzeitig mit 25 %iger wäßriger Ammoniaklösung bei 20 °C unter Einhaltung eines pH-Wertes von 9 in eine Vorlage einlaufen. Nach 2-stündigem Nachrühren unter Einhaltung des pH-Wertes wird die Ausbeute an Nitronaphthalin-sulfonsäuren in der wäßrigen Reaktionslösung durch Hochdruckflüssigkeitschromatographie unter Verwendung eines sauren Elutionsmittels bestimmt. Nachstehend sind die Ausbeuten an Nitronaphthalin-sulfonsäuren und — zum Vergleich — die Ausbeuten in der nur mit Calciumcarbonat auf einen pH-Wert von 2 eingestellten Reaktionslösung zusammengestellt.

| | Base | Ausbeute bezogen auf jeweilige Naphthalin-trisulfonsäure (Mol.-%) |
|---|---|---|
| 1-Nitronaphthalin-3,6,8-trisulfonsäure | keine | 82,5 |
| | Ammoniak | 95,1 |
| 1-Nitronaphthalin-4,6,8-trisulfonsäure | keine | 44,4 |
| | Ammoniak | 68,9 |
| Naphthalin-1,3,5-trisulfonsäure | keine | 1,96 |
| | Ammoniak | 3,60 |
| Naphthalin-1,3,6-trisulfonsäure | keine | 0,28 |
| | Ammoniak | 0,30 |

Beispiel 14

150 ml 98 %iger Salpetersäure werden in einer Nitrierapparatur vorgelegt. In diese werden unter Kühlen mit Trockeneis/Aceton bei – 20 bis – 15 °C im Verlaufe von 40 Minuten 107 g (0,15 Mol) Naphthalin-1.3.5-trisulfonsäure (Zusammensetzung : 51,6 % Naphthalin-1.3.5-trisulfonsäure, 29,5 % Schwefelsäure, 19 % Wasser) eingetragen. Die Reaktionsmischung wird 90 Minuten bei – 20 bis – 15 °C nachgerührt und anschließend unter Kühlen bei 0 bis 5 °C auf etwa 1 000 g Eis gegeben. Die Ausbeuten an Nitronaphthalinsulfonsäuren in der so erhaltenen wäßrigen Reaktionslösung werden durch Hochdruckflüssigkeitschromatographie unter Verwendung eines sauren Elutionsmittels bestimmt.

Die vorstehend beschriebene, verdünnte, wäßrige Nitriergemischlösung wird bei 0 bis 5 °C durch Zugabe von 25 %iger wäßriger Ammoniaklösung auf einen pH-Wert von 9,5 bis 10 eingestellt und 5 Stunden bei diesem pH-Wert gerührt. Anschließend wird die Ausbeute an Nitronaphthalin-sulfonsäure in der so erhaltenen Reaktionslösung durch Hochdruckflüssigkeitschromatographie unter Verwendung eines sauren Elutionsmittels bestimmt. Nachstehend sind die Ausbeuten an Nitronaphthalin-sulfonsäure in der sauren Nitriergemischlösung und der mit Ammoniak behandelten Reaktionslösung zusammengestellt.

| | Base | Ausbeute (Mol.-%) |
|---|---|---|
| 1-Nitronaphthalin-4,6,8-trisulfonsäure | keine | 39,6 |
| | Ammoniak | 86 |
| 2-Nitronaphthalin-4,6,8-trisulfonsäure | keine | 8,5 |
| | Ammoniak | 10,5 |
| Naphthalin-1,3,5-trisulfonsäure | keine | 0,1 |
| | Ammoniak | 0,7 |

Beispiel 15

Naphthalin-1.3.5-trisulfonsäure wird wie in Beispiel 14 beschrieben nitriert. Vor dem Verdünnen des Nitriergemisches mit Eis wird jedoch die überschüssige Salpetersäure im Hochvakuum bei – 20 bis – 10 °C innerhalb von 4 Stunden abdestilliert. Die Ausbeuten an Nitronaphthalin-sulfonsäuren werden wie in Beispiel 14 sowohl in der sauren Nitriergemischlösung als auch in der nach der Ammoniakbehandlung angefallenen wäßrigen Reaktionslösung durch Hochdruckflüssigkeitschromatographie unter Verwendung eines sauren Elutionsmittels bestimmt. Die Ausbeuten sind nachstehend zusammengestellt.

| | Base | Ausbeute (Mol.-%) |
|---|---|---|
| 1-Nitronaphthalin-4,6,8-trisulfonsäure | keine | 41 |
| | Ammoniak | 85 |
| 2-Nitronaphthalin-4,6,8-trisulfonsäure | keine | 7 |
| | Ammoniak | 11 |
| Naphthalin-1,3,5-trisulfonsäure | keine | 0,2 |
| | Ammoniak | 1,5 |

Beispiel 16

(Vergleichsbeispiel ; Nitrierung gemäß F.I.A.T. Final Report, n° 1016, Seiten 42-44)

In einer Nitrierapparatur werden 381 g eines gemäß F.I.A.T. Final Report n° 1016, Seiten 42-44 hergestellten Naphthalintrisulfonierungsgemisches vorgelegt.

Das Gemisch wies folgende Zusammensetzung auf :

33,1 % Naphthalin-1.3.5-trisulfonsäure
10,8 % Naphthalin-1.3.6-trisulfonsäure
2,2 % Naphthalin-1.3.7-trisulfonsäure
Rest Schwefelsäure und überschüssiges $SO_3$
15,0 % Kohlenstoff

Das Sulfiergemisch wird bei 60 °C mit 25 g Wasser verdünnt, bei 30 bis 35 °C innerhalb von 12 Stunden mit 43 g (= 0,587 Mol Salpetersäure) Mischsäure (Zusammensetzung : 86 % Salpetersäure, 12 % Schwefelsäure, 2 % Wasser) nitriert und 1 Stunde bei 40 °C nachgerührt.

Von diesem Nitriergemisch wird eine Probe auf Eis gegeben und die Ausbeute an Nitronaphthalinsulfonsäuren in dieser verdünnten sauren Nitriergemischlösung mittels Hochdruckflüssigkeitschromatographie unter Verwendung eines sauren Elutionsmittels bestimmt.

Ein Teil dieser verdünnten sauren Nitriergemischlösung wird mit 25 %iger wäßriger Ammoniaklösung auf einen pH-Wert von 9 eingestellt. In dieser Lösung wird ebenfalls die Ausbeute an (Nitro)-naphthalinsulfonsäuren mittels Hochdruckflüssigkeitschromatographie unter Verwendung eines sauren Elutionsmittels bestimmt.

Die Hauptmenge des Nitriergemisches wird gemäß F.I.A.T. Final Report n° 1016, Seite 42-44 in Wasser eingetragen. Die entstehende Lösung wird von den nitrosen Gasen befreit und anschließend mit Calciumcarbonat neutralisiert. Nach dem Abfiltrieren des Gipses wird die Ausbeute an Naphthalinsulfonsäuren in der wäßrigen Lösung mittels Hochdruckflüssigkeitschromatographie unter Verwendung eines sauren Elutionsmittels bestimmt.

Die Ausbeuten an Nitro-naphthalin-sulfonsäuren in den verschiedenen Lösungen sind in Tabelle 5 zusammengestellt.

Tabelle 5

| | Base | pH-Wert | Ausbeute [Mol.-%] bezogen auf jeweilige Naphthalinsulfonsäure | Ausbeute [Mol.-%] bezogen auf eingesetztes Naphthalin |
|---|---|---|---|---|
| 1-Nitronaphthalin-3,6,8-trisulfonsäure | keine | < 1 | 94,5 | 21,1 |
| | Ammoniak | 9 | 94,8 | 21,2 |
| | $CaCO_3$ | | 93,6 | 21,0 |
| 1-Nitronaphthalin-4,6,8-trisulfonsäure | keine | < 1 | 45,2 | 30,9 |
| | Ammoniak | 9 | 48,1 | 32,9 |
| | $CaCO_3$ | | 45,6 | 31,2 |
| Naphthalin-1,3,5-trisulfonsäure | keine | < 1 | 14,8 | 10,2 |
| | Ammoniak | | 15,1 | 10,3 |
| | $CaCO_3$ | | 18,7 | 12,8 |
| Naphthalin-1,3,6-trisulfonsäure | keine | < 1 | 0,6 | 0,13 |
| | Ammoniak | 9 | 0,5 | 0,11 |
| | $CaCO_3$ | | 0,5 | 0,11 |

Beispiel 17

In einer Nitrierapparatur werden 903 g eines Nitriergemisches vorgelegt, das durch Nitrieren von 0,5 Mol reiner Naphthalin-1.5-disulfonsäure gemäß dem in der US-PS 2 191 820 beschriebenen Verfahren erhalten wurde. Das Nitriergemisch wird bei 20 °C mit 65 g Wasser verdünnt, auf 10 °C abgekühlt und unter Rühren und Kühlen innerhalb von 10 Minuten bei 10 °C mit 64,3 g (1,0 Mol) 98 %iger Salpetersäure versetzt. Die Reaktionsmischung wird 170 Minuten bei 10 °C nachgerührt und anschließend in 300 g Eis eingetragen, wobei eine Temperatur von 10 °C nicht überschritten wird.

Die Zusammensetzung der auf diese Weise erhaltenen verdünnten sauren Nitriergemischlösung wird mittels Hochdruckflüssigkeitschromatographie unter Verwendung eines sauren Elutionsmittels bestimmt. Die verdünnte saure Nitriergemischlösung und 25 %ige wäßrige Ammoniaklösung werden

gleichzeitig bei 20 bis 25 °C und unter Einhaltung eines pH-Wertes von 9 in eine Vorlage eingetragen und der pH-Wert von 9 vier Stunden lang konstant gehalten.

Die Reaktionsmischung wird anschließend mit Schwefelsäure auf einen pH-Wert von 4 eingestellt. Nach mehrstündigem Stehen wird das ausgefallene Produkt abfiltriert, mit gesättigter Ammoniumsulfatlösung gewaschen und im Vakuum bei 50 °C getrocknet. Ausbeute 187 g trockenes Produkt. Es enthält 55,5 % 1,6-Dinitronaphthalin-4,8-disulfonsäure, das sind 54,9 %, bezogen auf eingesetzte Naphthalin-1,5-disulfonsäure.

Nachstehend sind die Ausbeuten an 3.8-Dinitronaphthalin-1.5-disulfonsäure in der verdünnten sauren Nitriergemischlösung und in der mit Ammoniak behandelten verdünnten Lösung angegeben.

| | Base | Ausbeute bezogen auf Naphthalin-1,5-disulfonsäure (Mol.-%) |
|---|---|---|
| 1,6-Dinitronaphthalin-4,8-disulfonsäure | keine | 33,8 |
| | Ammoniak | 60,9 |

Beispiel 18

Die in Beispiel 17 beschriebene Nitrierreaktion wird wiederholt, mit der Abänderung, daß das gemäß US-PS 2 191 820 erhaltene Nitriergemisch in Gegenwart von 66,1 g (0,5 Mol) Ammoniumsulfat bei Temperaturen von 30 °C innerhalb von 10 Minuten nitriert und das Nitriergemisch 80 Minuten bei 30 °C nachgerührt wird.

Es werden 173,2 g trockenes Produkt mit einem Gehalt an 1.6-Dinitronaphthalin-4.8-disulfonsäure von 66 % (60,48 % bezogen auf Naphthalin-1,5-disulfonsäure) erhalten.

Nachstehend sind die Ausbeuten an 1.6-Dinitronaphthalin-4.8-disulfonsäure in der verdünnten sauren Nitriergemischlösung und in der mit Ammoniak behandelten verdünnten wäßrigen Reaktionslösung angegeben.

| | Base | Ausbeute bezogen auf Naphthalin-1,5-disulfonsäure (Mol.-%) |
|---|---|---|
| 1,6-Dinitronaphthalin-4,8-disulfonsäure | keine | 33,52 |
| | Ammoniak | 64,83 |

Beispiel 19

(Vergleichsbeispiel ; Nitrierung gemäß DE-PS 72 665)

2-Nitronaphthalin-4.8-disulfonsäure wird in Schwefelsäure mit Salpetersäure bei 90 °C wie in der DE-PS 72 665 beschrieben nitriert. Nach 85-stündiger Reaktionsdauer beträgt die Ausbeute an 1.6-Dinitronaphthalin-4.8-disulfonsäure 48 %, bezogen auf eingesetzte 2-Nitronaphthalin-4.8-disulfonsäure.

Zum Vergleich wird die mit Wasser verdünnte saure Nitriergemischlösung mit 25 %iger wäßriger Ammoniaklösung auf einen pH-Wert von 9 eingestellt und 4 Stunden bei diesem pH-Wert gehalten. Die Ausbeute an 1.6-Dinitronaphthalin-4.8-disulfonsäure liegt unverändert bei 48 % der Theorie.

Beispiel 20

16,2 g (0,05 Mol) Naphthalin-1,5-disulfonsäure (Diammoniumsalz) werden unter Rühren bei − 10 bis − 20 °C in 289 g 90 %ige Schwefelsäure eingetragen. Die Mischung wird anschließend innerhalb von 6 Minuten mit 3,86 g 98 %iger Salpetersäure versetzt. Nach 6-minütigem Rühren wird die Reaktionslösung auf 435 g Eis gegossen.

Von der so erhaltenen wässrigen sauren Lösung wird eine Probe entnommen (Probe 1). Die übrige Lösung wird unter Rühren und starkem Kühlen bei Temperaturen zwischen 0 und 25 °C mit 444 ml 25 %iger Ammoniaklösung auf einen pH-Wert von 9 eingestellt, 2 Stunden bei 20 °C aufbewahrt und anschließend mit 17 ml 50 %iger Schwefelsäure auf einen pH-Wert von 3 eingestellt. Auch von dieser Lösung wird eine Probe entnommen (Probe 2).

Die Zusammensetzung der beiden Proben 1 und 2 wird mittels Hochdruckflüssigkeitschromatographie unter Verwendung eines sauren Elutionsmittels bestimmt. Es werden folgende Ausbeuten (in

Mol.-%) bezogen auf eingesetztes Naphthalin-1,5-disulfonsäure-diammoniumsalz gefunden :

| Probe 1 | in Probe 2 | |
|---|---|---|
| 8,56 | 8,34 | Naphthalin-1,5-disulfonsäure |
| 43,29 | 66,16 | 1-Nitronaphthalin-4,8-disulfonsäure |
| 26,76 | 27,26 | 2-Nitronaphthalin-4,8-disulfonsäure |

**Ansprüche**

1. Verfahren zur Herstellung von Nitronaphthalin-sulfonsäuren der Formel (I)

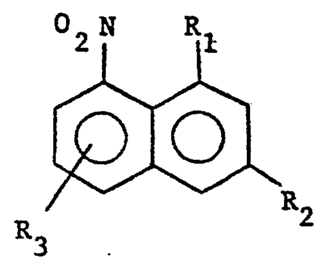

(I)

in der
$R_3$ in 5- oder 6-Stellung steht,
$R_1$ und $R_3$ unabhängig voneinander für eine $SO_3H$- oder $NO_2$-Gruppe stehen,
$R_2$ für ein Wasserstoffatom, eine $SO_3H$- oder $NO_2$-Gruppe steht, mit der Maßgabe, daß mindestens einer der Substituenten $R_1$, $R_2$ oder $R_3$ eine $SO_3H$-Gruppe ist, durch Umsetzen der entsprechenden Naphthalinsulfonsäuren der allgemeinen Formel (II)

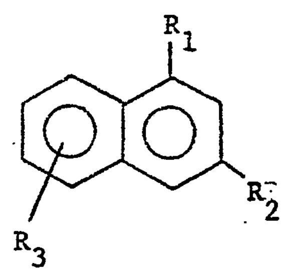

(II)

in der
$R_1$, $R_2$ und $R_3$ die vorstehend angegebene Bedeutung haben, mit Salpetersäure und Aufarbeiten des Nitriergemisches, dadurch gekennzeichnet, daß man die Umsetzung der Naphthalinsulfonsäuren mit Salpetersäure abbricht, wenn mehr als 90 % der eingesetzten Naphthalinsulfonsäuren umgesetzt sind, aber die Nitrierreaktion noch nicht beendet ist und das Nitriergemisch, gegebenenfalls nach Verdünnen mit Wasser, mit Basen bei einem pH-Wert von 5 bis 14 behandelt.

2. Verfahren nach Anspruch 1, dadurch gekennzeichnet, daß man bei der Umsetzung von Naphthalin-1.3.6-trisulfonsäure mit Salpetersäure die Nitrierreaktion abbricht, sobald mehr als 90 % der eingesetzten Naphthalin-1.3.6-trisulfonsäure umgesetzt sind und die Ausbeute an 1-Nitronaphthalin-3.6.8-trisulfonsäure im Nitriergemisch höchstens 90 % der theoretischen, auf Naphthalin-1.3.6-trisulfonsäure bezogenen Ausbeute beträgt.

3. Verfahren nach Anspruch 1, dadurch gekennzeichnet, daß man bei der Umsetzung von Naphthalin-1.3.5-trisulfonsäure mit Salpetersäure die Nitrierreaktion abbricht, sobald mehr als 90 % der eingesetzten Naphthalin-1.3.5-trisulfonsäure umgesetzt sind und die Ausbeute an 1-Nitronaphthalin-4.6.8-trisulfonsäure im Nitriergemisch höchstens 50 % der theoretischen, auf Naphthalin-1.3.5-trisulfonsäure bezogenen Ausbeute beträgt.

4. Verfahren nach Anspruch 1, dadurch gekennzeichnet, daß man bei der Umsetzung von 2-Nitronaphthalin-4.8-disulfonsäure mit Salpetersäure die Nitrierreaktion abbricht, sobald mehr als 90 % der eingesetzten 2-Nitronaphthalin-4.8-disulfonsäure umgesetzt sind und die Ausbeute an 1.6-Dinitronaphthalin-4.8-disulfonsäure im Nitriergemisch höchstens 50 % der theoretischen, auf 2-Nitronaphthalin-4.8-disulfonsäure bezogenen Ausbeute beträgt.

5. Verfahren nach Ansprüchen 1 bis 4, dadurch gekennzeichnet, daß man die Umsetzung der Naphthalinsulfonsäure mit Salpetersäure in 85 bis 95 gew.-%iger Schwefelsäure bei Temperaturen von 0 bis 50 °C durchführt und je Mol Naphthalinsulfonsäure 1,05 bis 2,5 Mol Salpetersäure einsetzt.

6. Verfahren nach Ansprüchen 1 bis 4, dadurch gekennzeichnet, daß man die Umsetzung der Naphthalinsulfonsäure mit Salpetersäure in überschüssiger 80 bis 100 gew.-%iger Salpetersäure bei Temperaturen von − 40 bis + 20 °C durchführt.

7. Verfahren nach Ansprüchen 1 bis 6, dadurch gekennzeichnet, daß die Umsetzung der Naphthalinsulfonsäuren mit Salpetersäure in Gegenwart von Alkalisulfaten durchgeführt wird.

8. Verfahren nach Ansprüchen 1 und 2 und 5 bis 7, dadurch gekennzeichnet, daß man das bei der Umsetzung von Naphthalin-1.3.6-trisulfonsäure mit Salpetersäure erhaltene, gegebenenfalls mit Wasser verdünnte, Nitriergemisch bei einem pH-Wert von 5 bis 10 und Temperaturen von − 10 bis + 130 °C mit anorganischen und/oder organischen Basen behandelt, bis der Gehalt an 1-Nitronaphthalin-3.6.8-trisulfonsäure in dem behandelten Nitriergemisch nicht mehr ansteigt.

9. Verfahren nach Ansprüchen 1 und 3 bis 7, dadurch gekennzeichnet, daß man das bei der Umsetzung von Naphthalin-1.3.5-trisulfonsäure oder 2-Nitronaphthalin-4.8-disulfonsäure mit Salpetersäureerhaltene, gegebenenfalls mit Wasser verdünnte Nitriergemisch bei einem pH-Wert von 5 bis 10 und Temperaturen von − 10 bis + 80 °C mit Ammoniak und/oder organischen Basen behandelt, bis der Gehalt an 1-Nitronaphthalin-4.6.8-trisulfonsäure bzw. 1.6-Dinitronaphthalin-4.8-disulfonsäure in dem behandelten Nitriergemisch nicht mehr ansteigt.

**Claims**

1. Process for the preparation of nitronaphthalene-sulphonic acids of the formula (I)

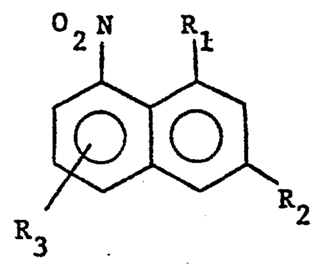

(I)

in which

$R_3$ is in the 5-position or 6-position,

$R_1$ and $R_3$ independently of one another represent a $SO_3H$ group or $NO_2$ group and

$R_2$ represents a hydrogen atom, or a $SO_3H$ or $NO_2$ group, with the proviso that at least one of the substituents $R_1$, $R_2$ or $R_3$ is a $SO_3H$ group, by reacting the corresponding naphthalenesulphonic acids of the general formula (II)

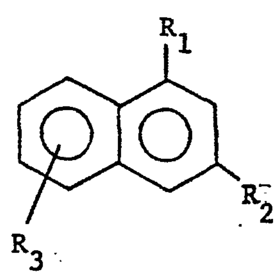

(II)

in which

$R_1$, $R_2$ and $R_3$ have the meaning given above, with nitric acid and working up the nitration mixture, characterised in that the reaction of the naphthalenesulphonic acids with nitric acid is terminated when more than 90 % of the naphthalenesulphonic acids employed have reacted but the nitration reaction has not yet ended and the nitration mixture, if appropriate after being diluted with water, is treated with bases at a pH value of 5 to 14.

2. Process according to Claim 1, characterised in that in the reaction of naphthalene-1,3,6-trisulphonic acid with nitric acid the nitration reaction is terminated as soon as more than 90 % of the naphthalene-1,3,6-trisulphonic acid employed has reacted, and the yield of 1-nitronaphthalene-3,6,8-trisulphonic acid in the nitration mixture is at most 90 % of the theoretical yield, relative to the naphthalene-1,3,6-trisulphonic acid.

3. Process according to Claim 1, characterised in that in the reaction of naphthalene-1,3,5-trisulphonic acid with nitric acid the nitration reaction is terminated as soon as more than 90 % of the naphthalene-1,3,5-trisulphonic acid employed has reacted and the yield of 1-nitronaphthalene-4,6,8-trisulphonic acid in the nitration mixture is at most 50 % of the theoretical yield, relative to the naphthalene-1,3,5-trisulphonic acid.

4. Process according to Claim 1, characterised in that in the reaction of 2-nitronaphthalene-4,8-disulphonic acid with nitric acid the nitration reaction is terminated as soon as more than 90 % of the 2-nitronaphthalene-4,8-disulphonic acid has reacted and the yield of 1,6-dinitronaphthalene-4,8-disulphonic acid in the nitration mixture is at most 50 % of the theoretical yield, relative to the 2-nitronaphthalene-4,8-disulphonic acid.

5. Process according to Claims 1 to 4, characterised in that the reaction of the naphthalenesulphonic acid with nitric acid is carried out in 85 to 95 % strength by weight sulphuric acid at temperatures of 0 to 50 °C and 1.05 to 2.5 mols of nitric acid are employed per mol of naphthalenesulphonic acid.

6. Process according to Claims 1 to 4, characterised in that the reaction of the naphthalenesulphonic acid with nitric acid is carried out in excess 80 to 100 % strength by weight nitric acid at temperatures of − 40 to + 20 °C.

7. Process according to Claims 1 to 6, characterised in that the reaction of the naphthalenesulphonic acids with nitric acid is carried out in the presence of alkali metal sulphates.

8. Process according to Claims 1 and 2 and 5 to 7, characterised in that the nitration mixture which is obtained in the reaction of naphthalene-1,3,6-trisulphonic acid with nitric acid and which has been diluted with water if necessary, is treated with inorganic and/or organic bases at a pH value of 5 to 10 and temperatures of − 10 to + 130 °C, until the content of 1-nitronaphthalene-3,6,8-trisulphonic acid in the treated nitration mixture no longer increases.

9. Process according to Claims 1 and 3 to 7, characterised in that the nitration mixture which is obtained in the reaction of naphthalene-1,3,5-trisulphonic acid or 2-nitronaphthalene-4,8-disulphonic acid with nitric acid and which has been diluted with water if necessary is treated with ammonia and/or organic bases at a pH value of 5 to 10 and at temperatures of − 10 to + 80 °C, until the content of 1-nitronaphthalene-4,6,8-trisulphonic acid or 1,6-dinitronaphthalene-4,8-disulphonic acid in the treated nitration mixture no longer increases.

**Revendications**

1. Procédé de préparation d'acides nitronaphtalène-sulfoniques de formule I

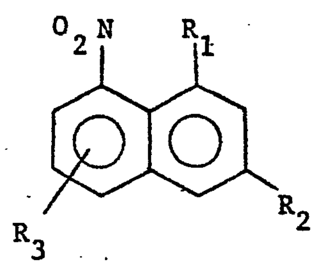

(I)

dans laquelle

$R_3$ est en position 5 ou 6,

$R_1$ et $R_3$ représentent chacun, indépendamment l'un de l'autre, un groupe $SO_3H$ ou $NO_2$,

$R_2$ représente un atome d'hydrogène, un groupe $SO_3H$ ou $NO_2$, étant spécifié que l'un au moins des symboles $R_1$, $R_2$ ou $R_3$ représente un groupe $SO_3H$, par réaction des acides naphtalène-sulfoniques correspondants de formule générale II

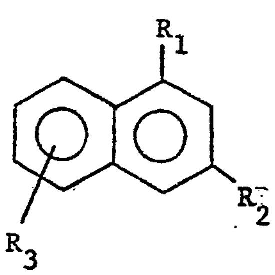

(II)

dans laquelle

$R_1$, $R_2$ et $R_3$ ont les significations indiquées ci-dessus, avec l'acide nitrique et traitement du mélange

de nitration, caractérisé en ce que l'on interrompt la réaction des acides naphtalène-sulfoniques avec l'acide nitrique lorsqu'on a converti plus de 90 % des acides naphtalène-sulfoniques mis en œuvre mais que la réaction de nitration n'est pas encore terminée, et on traite le mélange de nitration, éventuellement après dilution par l'eau, à l'aide de bases à un pH de 5 à 14.

2. Procédé selon la revendication 1, caractérisé en ce que, dans le cas de la réaction de l'acide naphtalène-1,3,6-trisulfonique avec l'acide nitrique, on interrompt la réaction de nitration dès qu'on a converti plus de 90 % de l'acide naphtalène-1,3,6-trisulfonique mis en œuvre et alors que le rendement en acide 1-nitronaphtalène-3,6,8-trisulfonique dans le mélange de nitration représente au maximum 90 % du rendement théorique par rapport à l'acide naphtalène-1,3,6-trisulfonique.

3. Procédé selon la revendication 1, caractérisé en ce que, dans le cas de la réaction de l'acide naphtalène-1,3,5-trisulfonique avec l'acide nitrique, on interrompt la réaction de nitration dès qu'on a converti plus de 90 % de l'acide naphtalène-1,3,5-trisulfonique mis en œuvre et alors que le rendement en acide 1-nitronaphtalène-4,6,8-trisulfonique dans le mélange de nitration représente au maximum 50 % du rendement théorique, par rapport à l'acide naphtalène-1,3,5-trisulfonique.

4. Procédé selon la revendication 1, caractérisé en ce que, dans le cas de la réaction de l'acide 2-nitronaphtalène-4,8-disulfonique avec l'acide nitrique, on interrompt la réaction de nitration dès qu'on a converti plus de 90 % de l'acide 2-nitro-naphtalène-4,8-disulfonique mis en œuvre et alors que le rendement en acide 1,6-dinitronaphtalène-4,8-disulfonique dans le mélange de nitration représente au maximum 50 % du rendement théorique, par rapport à l'acide 2-nitronaphtalène-4,8-disulfonique.

5. Procédé selon les revendications 1 à 4, caractérisé en ce que la réaction de l'acide naphtalène-sulfonique avec l'acide nitrique est effectuée dans l'acide sulfurique à une concentration de 85 à 95 % en poids à des températures de 0 à 50 °C et en utilisant 1,05 à 2,5 moles d'acide nitrique par mole d'acide naphtalène-sulfonique.

6. Procédé selon les revendications 1 à 4, caractérisé en ce que la réaction de l'acide naphtalène-sulfonique avec l'acide nitrique est effectuée dans un excès d'acide nitrique à une concentration de 80 à 100 % en poids à des températures de − 40 à + 20 °C.

7. Procédé selon les revendications 1 à 6, caractérisé en ce que la réaction des acides naphtalène-sulfoniques avec l'acide nitrique est effectuée en présence de sulfates alcalins.

8. Procédé selon les revendications 1 et 2 et 5 à 7, caractérisé en ce que le mélange de nitration obtenu à la réaction de l'acide naphtalène-1,3,6-trisulfonique avec l'acide nitrique, éventuellement dilué par l'eau, est traité par des bases minérales et/ou organiques à un pH de 5 à 10 et à des températures de − 10 à + 130 °C jusqu'à ce que la teneur en acide 1-nitronaphtalène-3,6,8-trisulfonique dans le mélange de nitration traité n'augmente plus.

9. Procédé selon les revendications 1 et 3 à 7, caractérisé en ce que le mélange de nitration obtenu à la réaction de l'acide naphtalène-1,3,5-trisulfonique ou de l'acide-2-nitronaphtalène-4,8-disulfonique avec l'acide nitrique, éventuellement dilué à l'eau, est traité par l'ammoniac et/ou des bases organiques à un pH de 5 à 10 et à des températures de − 10 à + 80 °C, jusqu'à ce que la teneur en acide 1-nitronaphtalène-4,6,8-trisulfonique ou 1,6-dinitronaphtalène-4,8-disulfonique respectivement dans le mélange de nitration traité n'augmente plus.